# EUROPEAN PATENT APPLICATION

(11) **EP 4 019 974 A1**
(43) Date of publication of application: **29.06.2022**
(21) Application number: 20383140.9
(22) Date of filing: 22.12.2020
(51) Int. Cl.: G01N 33/569

(54) **IN VITRO METHOD FOR PREDICTING MORTALITY IN COVID-19 PATIENTS**

(71) Applicant: Fundación Instituto de Estudios Ciencias de la Salud de Castilla y León (IECSCYL-IBSAL), 37007 Salamanca (ES); Kelvin, David Joseph, Halifax, NS B3N 3B9 (CA); Gerencia Regional de Salud de Castilla y León, 47007 Valladolid (ES); Fundación para la Investigación Biomédica del Hospital Gregorio Marañón (FIBHGM), 28007 Madrid (ES); Universidad de Valladolid, 47002 Valladolid (ES); Consorcio Centro de Investigación Biomédica en Red, M.P. (CIBER), 28029 Madrid (ES); Institut d'Investigacions Biomèdiques August Pi i Sunyer (IDIBAPS), 08036 Barcelona (ES); Universitat de Barcelona, 08028 Barcelona (ES); Hospital Clínic de Barcelona, 08036 Barcelona (ES)
(72) Inventor: BERMEJO MARTÍN, Jesús Francisco, 37007 Salamanca (ES); ALMANSA MORA, Raquel, 37007 Salamanca (ES); SANTOS TEDIM SOUSA PEDROSA, Ana Sofía, 37007 Salamanca (ES); BUSTAMANTE MUNGUIRA, Elena, 47005 Valladolid (ES); TAMAYO LOMAS, Luis Mariano, 47012 Valladolid (ES); ALDECOA ÁLVAREZ- SANTULLANO, César, 47012 Valladolid (ES); DOMINGUEZ-GIL GONZALEZ, Marta, 47012 Valladolid (ES); EIROS BOUZA, Jose María, 47012 Valladolid (ES); KELVIN, David Joseph, Nova Scotia, Nova Scotia B3N 3B9 (CA); BARBÉ ILLA, Ferrán, 25198 Lleida (ES); TORRES MARTÍ, Antoni, 08036 Barcelona (ES); MICHELOUD GIMÉNEZ, Dariela Edhit, 28007 Madrid (ES); GÓMEZ GARCÍA, Jose Manuel, 28007 Madrid (ES); GONZÁLEZ RIVERA, Milagros, 28007 Madrid (ES)
(74) Representative: Hoffmann Eitle

(57) **Abstract**

The present invention refers to the *in vitro* use of a Coronavirus structural protein, measured in plasma, serum or blood samples obtained from the patient, for the prognosis and/or for predicting the mortality risk of patients suffering from Coronavirus infection, for determining the presence of viremia and/or high degree of viral replication in patients suffering from Coronavirus infection, or for selecting patients suffering from Coronavirus infection for receiving an antiviral therapy.

## Description

### FIELD OF THE INVENTION

The present invention refers to the medical field. Particularly, it refers to the *in vitro* use of a Coronavirus structural protein, measured in plasma, serum or blood samples obtained from the patient, for the prognosis and/or for predicting the mortality risk of patients suffering from Coronavirus infection, for determining the presence of viremia and/or high degree of viral replication in patients suffering from Coronavirus infection, or for selecting patients suffering from Coronavirus infection for receiving an antiviral therapy.

### STATE OF THE ART

A week after alerting the WHO of a cluster of pneumonia of unknown etiology in Wuhan, the Chinese authorities announced on 7 January 2020 that a novel coronavirus was identified as the cause of these pneumonia. According to phylogenetic analysis, this novel severe acute respiratory syndrome coronavirus 2 (SARS-CoV-2), previously named 2019-nCoV, belongs to the B lineage of Betacoronavirus genus and the Sarbecovirus subgenus and has more than 85% nucleotide sequence identity with a bat SARS-like CoV genome published previously. On January 30, WHO declared COVID-19 outbreak a public health emergency of international concern and the disease has now spread worldwide.

In this context, highly sensitive and specific tests are crucial to identify and manage COVID-19 patients and implement control measures to limit the outbreak. Real time reverse transcription polymerase chain reaction (RT-qPCR) in respiratory samples is the current recommended laboratory method to diagnose SARS-CoV-2 acute infection. However, performing RT-qPCR requires special equipment and skilled laboratory personnel familiar with molecular techniques. Moreover, molecular tests are costly and often time consuming.

Consequently, test designed to detect SARS-CoV-2 antigen in nasopharyngeal secretions have been developed and have been included in the first line of diagnostic tests for COVID-19.

However, there is still an unmet medical need of finding highly sensitive and specific tests aimed not only at diagnosing SARS-CoV-2 infection but also being able of predicting mortality risk, determining the presence of viremia and/or high degree of viral replication. This type of test would bring the opportunity of rapidly identifying and selecting patients with a higher mortality risk for receiving an antiviral therapy. In fact, critically ill patients, who could benefit the most from antiviral treatment, would be identified at an early state and treated accordingly.

The present invention is focused on solving the above indicated problem, and a test designed to detect SARS-CoV-2 antigen is herein provided, which can be used to predict mortality risk and determining the presence of viremia and/or high degree of viral replication.

### DESCRIPTION OF THE INVENTION

### Brief description of the invention

As explained above, the present invention refers to the *in vitro* use of a Coronavirus structural protein, measured in plasma, serum or blood samples obtained from the patient, for the prognosis and/or for predicting the mortality risk of patients suffering from Coronavirus infection, for determining the presence of viremia and/or high degree of viral replication in patients suffering from Coronavirus infection, or for selecting patients suffering from Coronavirus infection for receiving an antiviral therapy.

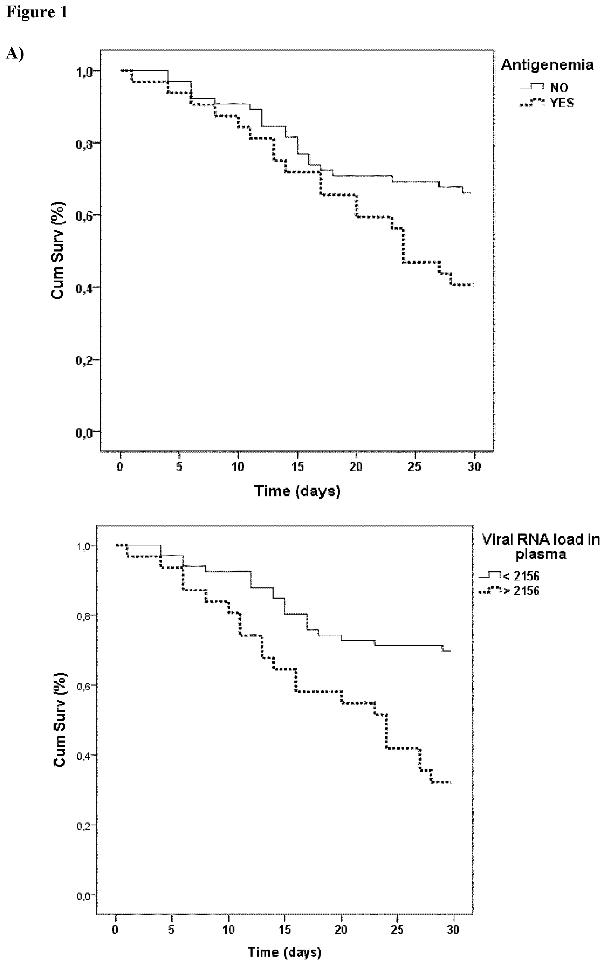

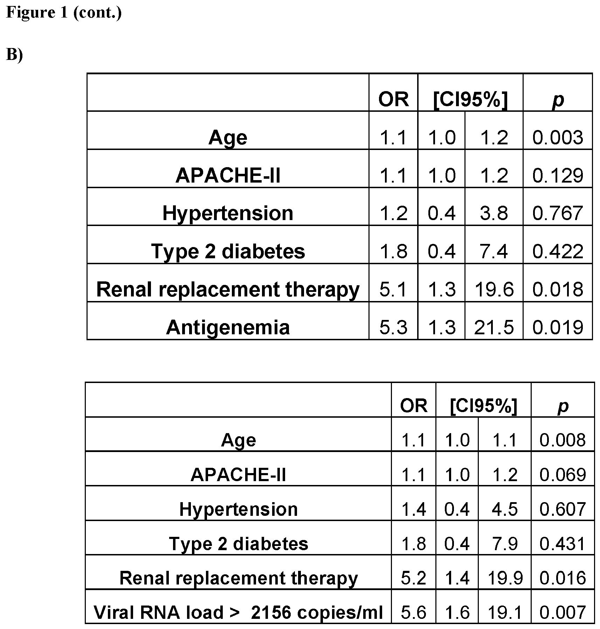

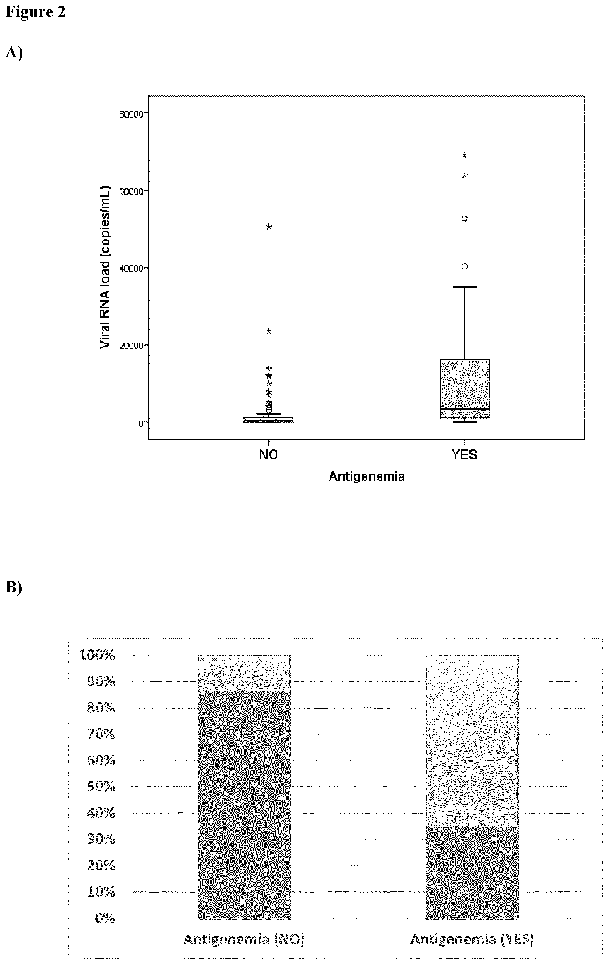

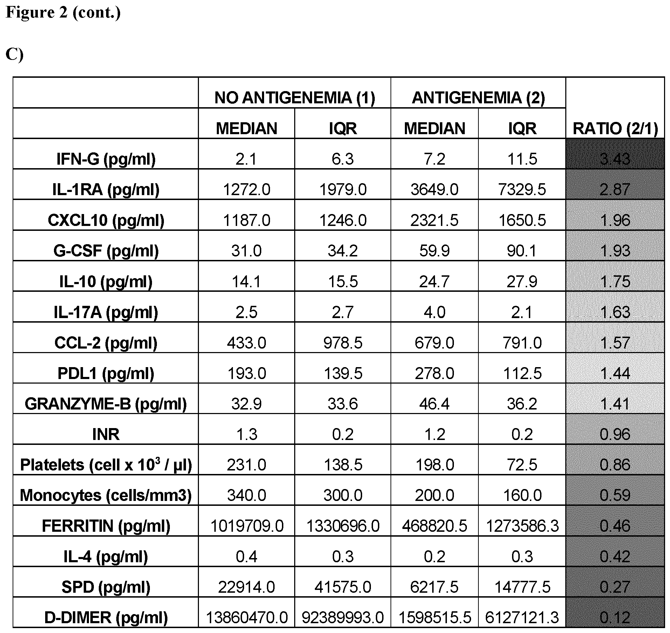

In this study, we employed a rapid test to detect SARS-CoV-2 antigen (Panbio COVID-19 rapid test device, Abbott, Illinois, United States) in plasma from critically ill COVID-19 patients, collected in the first 24 hours following admission to the ICU. 100 ul of plasma were diluted with 100 ul of the test buffer and loaded into the test device. Fifteen minutes later, the test result was read as positive or negative for the presence of antigen. As showed by the Kaplan Meier curves, patients with viral antigen in plasma died earlier than those with no antigen in plasma (**Figure 1**). In the multivariate logistic regression analysis, the presence of viral antigen in plasma translated into a 5-fold risk of dying in the first 30 days following admission to the ICU (**Figure 1**). A similar odds ratio was found for the presence of viral RNA load in plasma higher than 2156 copies/ml (**Figure 1**). The reason why antigenemia is associated to poor outcome is unclear, but further evidence suggests that patients who present viral antigen in blood are not able to control viral replication: 1) patients with antigenemia showed higher viral RNA loads in plasma than those with no antigenemia (as quantified by using droplet digital PCR) (**Figure 2**); 2) patients with antigenemia showed a lower frequency of anti-SARS-CoV-2 IgG (**Figure 2**); 3) these patients showed in addition higher levels of chemokines increasing in the context of active viral replication (CXCL10, CCL2), of molecules inducing immunosuppression (IL-1RA, IL-10 and PD-L1), lower levels of the antiviral molecule SPD, lower monocyte counts, and of the antibody inducer cytokine IL-4 (**Figure 2**). Finally, patients with antigenemia showed lower concentration of ferritin in plasma, and lower D-dimers and INR, with (paradoxically), lower platelet counts (**Figure 2**), findings which biological implications remains to be elucidated.

Our results demonstrate that detecting the presence of viral antigen in plasma using these fast tests informs on the probability of death of COVID-19 patients, making these tests a valuable tool to better select those patients to be admitted to the ICU, and helping to individualise treatment. Our results demonstrate also that he presence of viral antigen in plasma is a surrogated marker of increased viral replication, which could help to identify those patients deserving treatments with antiviral activity (drugs, monoclonal antibodies, hyperimmune serum i.e).

So, the first embodiment of the present invention refers to an *in vitro* method for the prognosis and/or for predicting mortality risk in patients suffering from Coronavirus infection, which comprises determining the presence or the level of a Coronavirus structural protein in plasma, serum or blood samples obtained from the patient, wherein the determination of the presence of a Coronavirus structural protein, or the quantification of a level of a Coronavirus structural protein statistically higher as compared with a pre-established threshold value (this would be the value measured in mildly infected patients which have not been hospitalized), is an indication of bad prognosis and/or of mortality risk.

The second embodiment of the present invention refers to an *in vitro* method for determining the existence of viremia and/or of high degree of viral replication in patients suffering from Coronavirus infection, which comprises determining the presence or the level of a Coronavirus structural protein in plasma, serum or blood samples obtained from the patient, wherein the determination of the presence of a Coronavirus structural protein, or the quantification of a level of a Coronavirus structural protein statistically higher as compared with a pre-established threshold value, is an indication of the presence of viremia and/or of high degree of viral replication.

The third embodiment of the present invention refers to an *in vitro* method for selecting patients suffering from Coronavirus infection for receiving an antiviral therapy, which comprises determining the presence or the level of a Coronavirus structural protein in plasma, serum or blood samples obtained from the patient, wherein if the presence of a Coronavirus structural protein is determined, or a statistically higher level of a Coronavirus structural protein is quantified as compared with a pre-established threshold value, the patient is elected for receiving an antiviral therapy.

The fourth embodiment of the present invention refers to the *in vitro* use of a Coronavirus structural protein in plasma, serum or blood samples obtained from the patient, for the prognosis and/or for predicting the mortality risk of patients suffering from Coronavirus infection, for determining the presence of viremia and/or viral replication in patients suffering from Coronavirus infection, or for selecting patients suffering from Coronavirus infection for receiving an antiviral therapy.

The fifth embodiment of the present invention refers to an antiviral composition for use in the treatment of those patients suffering from Coronavirus infection in which the presence of a Coronavirus structural protein has been determined, or a level of a Coronavirus structural protein statistically higher as compared with a pre-established threshold value has been quantified, in plasma, serum or blood samples of the patient.

In a preferred embodiment, the present invention refers to an antiviral composition for use in the treatment of patients suffering from Coronavirus infection, which comprises: (i) determining in plasma, serum or blood samples the presence or the level of a Coronavirus structural protein and (ii) administering to the patient an effective amount of the antiviral composition if the presence of a Coronavirus structural protein is detected or a level of a Coronavirus structural protein statistically higher as compared with a pre-established threshold value has been quantified.

Equally, the present invention also refers to a method for treating patients suffering from Coronavirus infection in which the presence of a Coronavirus structural protein has been determined, or a level of a Coronavirus structural protein statistically higher as compared with a pre-established threshold value has been quantified, in plasma, serum or blood samples, which comprises the administration of a therapeutically effective amount of an antiviral composition.

In a preferred embodiment, the Coronavirus infection is caused by SARS-CoV-2 and the structural protein detected is selected from: nucleocapsid (N), Spike (S), Membrane (M) and/or Envelope (E) proteins, most preferably nucleocapsid (N).

In a preferred embodiment, the above methods are characterized in that the presence or the level of the structural protein is measured by using mass spectrometry or an immunoassay, preferably ELISA, immunochromatography, nephelometry, Luminex, SimplePlex, or any other technique based on the antigen-antibody reaction.

The sixth embodiment of the present invention refers to a kit-of-parts for performing any of the methods described above which comprises:
a. Means for obtaining plasma, serum or blood samples from the patient.
b. Antibodies which specifically bind a structural protein of the Coronavirus, preferably a structural protein of the SARS-CoV-2 selected from: nucleocapsid (N), Spike (S), Membrane (M) and/or Envelope (E) proteins, preferably the nucleocapsid (N) protein of SARS-CoV-2, most preferably nucleocapsid (N).

In a preferred embodiment the kit-of-parts is an immunoassay, preferably ELISA, immunochromatography, nephelometry, Luminex, SimplePlex, or any other technique based on the antigen-antibody reaction.

For the purpose of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of' means including, and it is limited to, whatever follows the phrase "consisting of'. Thus, the phrase "consisting of' indicates that the listed elements are required or mandatory, and that no other elements may be present.
- By "therapeutically effective dose or amount" is intended an amount that, when administered as described herein, brings about a positive therapeutic response in a subject suffering a Coronavirus infection. The exact amount required will vary from subject to subject, depending on the age, and general condition of the subject, the severity of the condition being treated, mode of administration, and the like. An appropriate "effective" amount in any individual case may be determined by one of ordinary skill in the art using routine experimentation, based upon the information provided herein.
- A reference control level is herein is mentioned, when referring to the level of the antigens observed in patients. The patient is likely to have a bad prognosis with a given sensitivity and specificity if the levels of the antigens in the patient are above said reference control level. A reference value can be a "threshold value" or a "cut-off" value. Typically, a "threshold value" or "cut-off value" can be determined experimentally, empirically, or theoretically. According to the present invention, the "threshold" value refers the level of structural proteins identified in mild infected patients which has not been hospitalized.
- In the context of the present invention the term "antigenemia" refers to the condition of having an antigen present in plasma, serum or blood samples. Particularly, the antigen is a structural protein of the SARS-CoV-2 selected from: nucleocapsid (N), Spike (S), Membrane (M) and/or Envelope (E) proteins, preferably the nucleocapsid (N) protein of SARS-CoV-2, most preferably nucleocapsid (N).

### Brief description of the figures

**Figure 1****. A)** Kaplan Meier curves for 30-day mortality. **B)** Multivariate logistic regression analysis for 30-day mortality.
**Figure 2****. A)** Viral RNA load in plasma (p<0.001). **B)** % of patients with anti-SARS-CoV-2 IgG (dark blue) (*p*<0.001). **C)** Laboratory parameters in patients with presence / absence of antigenemia (p<0.05).

### Detailed description of the invention

The present invention is illustrated by means of the Examples set below without the intention of limiting its scope of protection.

### Example 1. Patient characteristics.

**Table 1** included below describes the cohort of critically ill patients suffering from COVID-19 who have been analysed in the present invention for the study of antigenemia and risk of mortality at 30 days.

### Example 2. Viral load analyses.

By using the Bio-Rad SARS-CoV-2 ddPCR kit (digital PCR), the best cut-off point for determining viral load has been established, by analysing the N1 fragment of the N gene of the virus. 1233 copies/mL in plasma of cDNA of the N1 fragment of the virus yield a sensitivity of 75% and a specificity of 75% to detect the presence of antigenemia. In other words, high levels viral load (or viral replication) are identified, with a sensitivity of 75% and a specificity of 75%, when more than 1233 copies of cDNA of the N1 fragment / mL are identified.

### Example 3. Antigenemia in plasma samples translates into an earlier morality.

Such as it is shown in **Figure 1** (**A**, upper) the present invention provides evidence that the presence of antigenemia in plasma samples translates into an earlier mortality, as assessed by the Kaplan Meier curves. This way, non-survivors showing antigenemia died 2 days earlier than those with no antigenemia. In addition, multivariate logistic regression analysis evidence that antigenemia was a predictor of mortality at 30 days following admission to the ICU, independently of major confounding variables **Figure 1** (**B**, upper). In turn, the presence of high levels of viral RNA in plasma **Figure 1** (**A**, lower), also translated into an earlier mortality, and constituted an independent risk factor for mortality, with a similar odds ratio than that of antigenemia **Figure 1** (**B**, lower). While quantifying viral load requires of complicated PCR-based methods, the methods described in the present invention to determine the presence of antigenemia are easier and faster to perform, since they are based in the antigen-antibody reaction.

### Example 4. Patients with antigenemia showed higher viral loads in plasma.

Such as it is shown in **Figure 2A**), patients with antigenemia showed higher viral loads in plasma, as assessed by using droplet digital PCR. As shown in **Figure 2B**), patients with antigenemia showed a lower frequency of specific IgG anti SARS-CoV-2, as assessed by the Abbott Architect SARS-CoV-2 IgG Assay (Illinois, U.S.A). According to **Figure 2C**), patients with antigenemia showed higher levels of several laboratory parameters indicating dysregulation of the host response to infection (indicated with red colour in the "ratio" column), and lower levels of other parameters indicating also dysregulation of the host response to infection (indicated with blue colour in the "ratio" column).

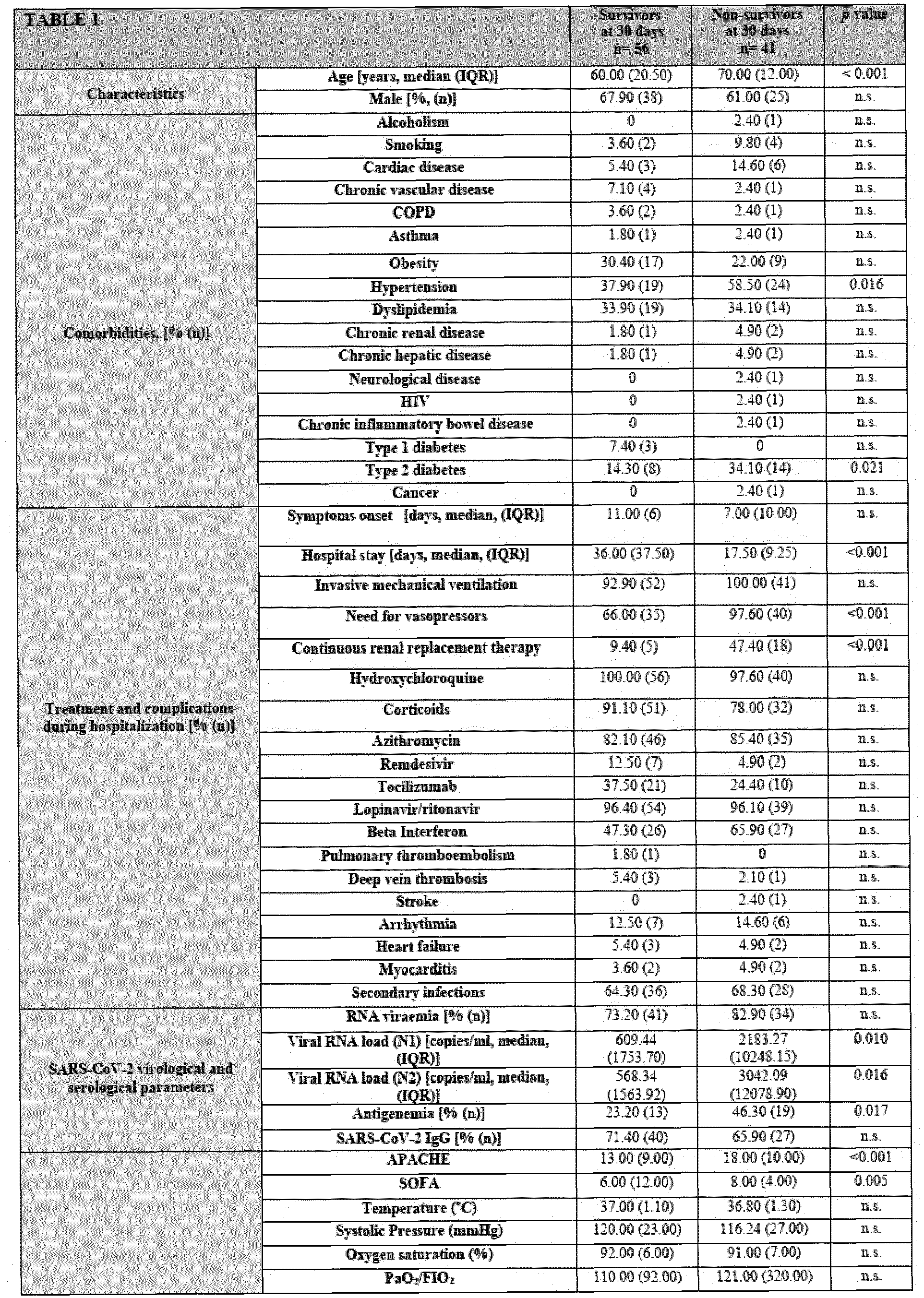

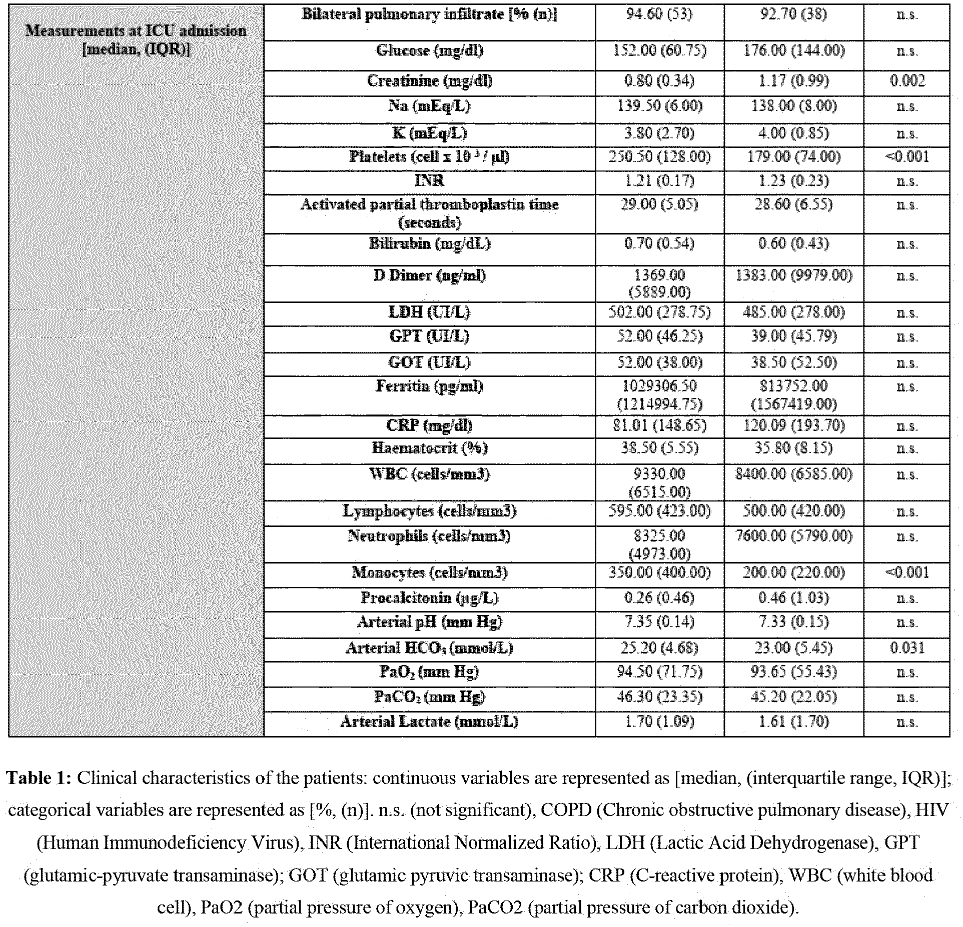

## Claims

1. *In vitro* method for the prognosis and/or for predicting mortality risk in patients suffering from Coronavirus infection, which comprises determining the presence or the level of a Coronavirus structural protein in plasma, serum or blood samples obtained from the patient, wherein the determination of the presence of a Coronavirus structural protein, or the quantification of a level of a Coronavirus structural protein statistically higher as compared with a pre-established threshold value, is an indication of bad prognosis and/or of mortality risk.

2. *In vitro* method for determining the existence of viremia and/or of high degree of viral replication in patients suffering from Coronavirus infection, which comprises determining the presence or the level of a Coronavirus structural protein in plasma, serum or blood samples obtained from the patient, wherein the determination of the presence of a Coronavirus structural protein, or the quantification of a level of a Coronavirus structural protein statistically higher as compared with a pre-established threshold value, is an indication of the presence of viremia and/or of high degree of viral replication.

3. *In vitro* method for selecting patients suffering from Coronavirus infection for receiving an antiviral therapy, which comprises determining the presence or the level of a Coronavirus structural protein in plasma, serum or blood samples obtained from the patient, wherein if the presence of a Coronavirus structural protein is determined, or a statistically higher level of a Coronavirus structural protein is quantified as compared with a pre-established threshold value, the patient is elected for receiving an antiviral therapy.

4. *In vitro* method, according to any of the previous claims, wherein the Coronavirus infection is caused by SARS-CoV-2 and the structural protein detected is selected from: nucleocapsid (N), Spike (S), Membrane (M) and/or Envelope (E) proteins.

5. *In vitro* method, according to any of the previous claims, **characterized in that** the presence or the level of the structural protein is measured by using mass spectrometry or an immunoassay, preferably ELISA, immunochromatography, nephelometry, Luminex, SimplePlex, or any other technique based on the antigen-antibody reaction.

6. *In vitro* use of a Coronavirus structural protein in plasma, serum or blood samples obtained from the patient, for the prognosis and/or for predicting the mortality risk of patients suffering from Coronavirus infection, for determining the presence of viremia and/or viral replication in patients suffering from Coronavirus infection, or for selecting patients suffering from Coronavirus infection for receiving an antiviral therapy.

7. *In vitro* use, according to claim 6, wherein the Coronavirus infection is caused by SARS-CoV-2 and the structural protein detected is selected from: nucleocapsid (N), Spike (S), Membrane (M) and/or Envelope (E) proteins.

8. Antiviral composition for use in the treatment of those patients suffering from Coronavirus infection in which the presence of a Coronavirus structural protein has been determined, or a level of a Coronavirus structural protein statistically higher as compared with a pre-established threshold value has been quantified, in plasma, serum or blood samples of the patient.

9. Antiviral composition for use in the treatment of patients suffering from Coronavirus infection, according to claim 8, which comprises: (i) determining in plasma, serum or blood samples the presence or the level of a Coronavirus structural protein and (ii) administering to the patient an effective amount of the antiviral composition if the presence of a Coronavirus structural protein is detected or a level of a Coronavirus structural protein statistically higher as compared with a pre-established threshold value has been quantified.

10. Antiviral composition for use in the treatment of patients suffering from Coronavirus infection, according to any of the claims 8 or 9, wherein the Coronavirus infection is caused by SARS-CoV-2 and the structural protein detected is selected from: nucleocapsid (N), Spike (S), Membrane (M) and/or Envelope (E) proteins.

11. kit-of-parts for performing any of the methods according to claims 1 to 5 which comprises:
a. Means for obtaining plasma, serum or blood samples from the patient.
b. Antibodies which specifically bind a structural protein of the Coronavirus.

12. kit-of-parts, according to claim 11, which comprises:
a. Means for obtaining plasma, serum or blood samples from the patient.
b. Antibodies which specifically bind a structural protein of the SARS-CoV-2 selected from: nucleocapsid (N), Spike (S), Membrane (M) and/or Envelope (E) proteins.

13. kit-of-parts, according to any of the claims 11 or 12, which comprises:
a. Means for obtaining plasma, serum or blood samples of the patient.
b. Antibodies which specifically bind the nucleocapsid (N) protein of SARS-CoV-2.

14. kit-of-parts, according to any of the claims 11 to 13, **characterized in that** it based on mass spectrometry or is an immunoassay.

15. kit-of-parts, according to any of the claims 11 to 14, **characterized in that** the immunoassay is ELISA, immunochromatography, nephelometry, Luminex, SimplePlex, or any other technique based on the antigen-antibody reaction.
